# EUROPEAN PATENT APPLICATION

(11) **EP 4 180 475 A1**
(43) Date of publication of application: **17.05.2023**
(21) Application number: 22206452.9
(22) Date of filing: 09.11.2022
(51) Int. Cl.: C08G 18/50, C07D 251/56, C08G 18/38, C08G 18/46

(54) **PROCESS FOR PRODUCING A TRIAZINE RING CONTAINING COMPOUND, ADDITION PRODUCT, TRIAZINE RING CONTAINING POLYOL, USE OF THE ADDITION PRODUCT, USE OF THE TRIAZINE RING CONTAINING POLYOL AND PU/PIR FOAM OBTAINED BY USING THE TRIAZINE RING CONTAINING POLYOL**

(30) Priority: 10.11.2021 WO PCT/EP2021/081209
(71) Applicant: Norchem Limited, 1913 Floriana (MT)
(72) Inventor: GNEDIN, Evgenij, 41747 Viersen (DE); SCHEGRAVIN, Kirill, 603124 Nizhny Novgorod (RU)
(74) Representative: TBK

(57) **Abstract**

The present invention relates to a process for producing a triazine ring containing compound, wherein the process comprises an addition reaction between a compound containing a triazine ring and at least two amino groups, and a Michael-acceptor, in the presence of a radical polymerization inhibitor, to obtain an addition product, wherein the process additionally comprises an esterification, transesterification, amidation or transamidation reaction between the addition product and a polyfunctional alcohol or a primary or secondary amino alcohol.

## Description

### Title of the invention

Process for producing a triazine ring containing compound, triazine ring containing polyol, use of an addition product, use of the triazine ring containing polyol and PU/PIR foam obtained by using the triazine ring containing polyol.

### Field of the invention

The present invention relates to a process for producing a triazine ring containing compound, a triazine ring containing polyol, the use of an addition product, the use of the triazine ring containing polyol and a PU/PIR foam obtained by using the triazine ring containing polyol.

### Background Art

Methods for the synthesis of triazine group containing polyols are known, but feature several drawbacks. A Mannich condensation of formaldehyde and amino alcohols as for example disclosed in CN 106883402 A requires the use of formaldehyde, a separate oxyalkylation step due to the final product having a very high viscosity, and has a narrow application field due to a very high reactivity of the product. A modification of the reaction product of melamine and formaldehyde as for example disclosed in CN 102504244 A requires many stages of the process, yields products with a short shelf life, high sensitivity towards acids and increased temperatures, and significant amounts of residual free formaldehyde and water. It is therefore difficult to obtain these products with low water contents. A polyalkylation of melamine as for example disclosed in CN 104877126 A requires the use of expensive solvents and special equipments, and features difficulties with solvent removal from the final product and solvent regeneration. A transamination of melamine as for example disclosed in DE 3422218 A1 requires a large excess of amine which has to be regenerated during the separation step and a high amount of acidic catalysts that need to be removed in the separation step. Further, ammonia is released during the process. An oxyalkylation of melamine as for example disclosed in US 4500655 A requires a large excess of carbonates, features low yields due to decomposition of cyclic carbonates under carbon dioxide release, and is very expensive due to highly expensive cyclic carbonates.

EP 3 303 446 A1 provides flame retardant polyesters obtainable by reacting an aromatic or aliphatic dicarboxylic acid and/or ester of anhydride thereof, with an aliphatic polyol or mixtures thereof and with melamine. Methods of making said polyesters are also provided.

CN 107602831 A relates to a production method for polyester polyol for hard foam. The production method comprises the steps of enabling 15-30 parts by mass of melamine and 70-85 parts by mass of dicarboxylic acid or anhydride to react for modification, and under the effect of a catalyst, performing condensation polymerization with polyalcohol or dicarboxylic acid or anhydride to prepare the polyester polyol for hard foam. Moreover, Patent Document 7 also provides a production method of the polyester polyol for hard foam, which has the characteristics of being environmentally friendly, efficient, low in toxicity and permanent; the produced structural flame retardant polyester polyol cannot maintain the flame retardant performance after being used for a long time, raw material price is low, relative cost advantage is obvious, and relatively high cost performance is achieved.

JP 2001/040075 A aims to obtain an aromatic polyester polyol for rigid polyurethane foam useful for production of polyurethane foam excellent in low smoke emission, flame retardance and high strength by reacting a polyalkylene terephthalate resin and a glycol. The objective aromatic polyester polyol comprising terephtahlic acid and a glycol component is obtained by reacting (A) a polyalkylene terephthalate resin and (B) a glycol (e.g. diethylene glycol), and has acid value ≤0.4 mgKOH/g and including ≤60 µg/g of antimony. The preferable hydroxyl value of the polyester polyol is 100-400 mgKOH/g, and the viscosity is 2,000-6,000 mPa/s at 25 °C.

WO 9629357 A1 relates to the use of 1,3,5-triazines which are substituted in the 2-, 4- and 6-position or tautomers thereof for controlling the crystallization of linear polyesters and/or polyester polyols. The 1,3,5-triazine may be, respectively, a cyanuric acid, thiocyanuric acid, melamine or a tautomeric form thereof, any of which may be substituted, and is prepared in situ during the preparation of the linear polyesters and/or polyester polyols.

MAGHSOODI SAMANEH ET AL: "Synthesis Nano Dendrimer Supramolecular with Melamine Core", ORIENTAL JOURNAL OF CHEMISTRY, [Online] vol. 32, no. 6, 25 December 2016 (2016-12-25), pages 2985-2992, ISSN: 0970-020X, DOI: 10.13005/ojc/320618; URL:http://dx.doi.org/10.13005/ojc/320618 discloses dendrimers, which are a family of three-dimensional polymers having nano dimension and are characterized by spherical structure. The dendrimers use melamine as core of the dendrimers. In this study, dendrimers were synthesized with melamine and methyl acrylate with ester and primary amine-terminated groups.

CN 105 622 955 A discloses a soil passivation restoration agent and a preparation method thereof.

US 2021/198305 A1 discloses oligonucleotide compositions and methods of use thereof.

US 2005/245711 A1 discloses self-photoinitiating water-dispersible acrylate ionomers and synthetic methods.

JP S63 286426 A discloses a melamine ring-containing (meth)acrylate prepolymer and a curable resin composition containing said prepolymer.

### Prior Art Documents

### Patent Documents

Patent Document 1: CN 106883402 A
Patent Document 2: CN 102504244 A
Patent Document 3: CN 104877126 A
Patent Document 4: DE 3422218 A1
Patent Document 5: US 4500655 A
Patent Document 6: EP 3 303 446 A1
Patent Document 7: CN 107602831 A
Patent Document 8: JP 2001/040075 A
Patent Document 9: WO 9629357 A1
Patent Document 10: MAGHSOODI SAMANEH ET AL: "Synthesis Nano Dendrimer Supramolecular with Melamine Core", ORIENTAL JOURNAL OF CHEMISTRY, [Online] vol. 32, no. 6, 25 December 2016 (2016-12-25), pages 2985-2992, ISSN: 0970-020X, DOI: 10.13005/ojc/320618; URL:http://dx.doi.org/10.13005/ojc/320618
Patent Document 11: CN 105 622 955 A
Patent Document 12: US 2021/198305 A1
Patent Document 13: US 2005/245711 A1
Patent Document 14: JP S63 286426 A

### Brief description of drawings

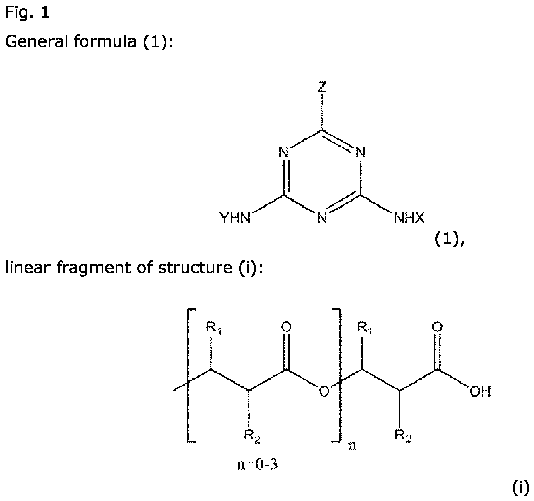

Fig. 1 describes a general formula of an addition product of a compound containing a triazine ring and at least two amino groups and a Michael-acceptor.

### Extended description of the invention

It is an object of the present invention to provide a triazine ring containing polyol having a long shelf life (i.e. polyol is stable and not sensitive to standard ambient conditions, i.e. stable for more than one year at 25°C and 100 kPa) and broad applicability in obtaining polyurethane/polyisocyanurate (PU/PIR) foams, preferably rigid PU/PIR foams, having improved flame retardancy properties (e.g. increased fire resistance and reduced smoke release). It is a further object to provide an improved production process for obtaining triazine ring containing polyols, which is simple (e.g. does not require any regeneration steps of reagents or solvents, does not implement any solvents, and does easily achieve a desired combination of viscosity and hydroxyl value in the final polyol) and does not require complicated equipment, preferably using equipment for production of polyester polyols or polyester resins. A further object is to use a triazine ring containing polyol in PU/PIR foams having improved flame retardancy properties.

The present invention provides the solutions as defined in the enclosed independent claims 1 and 10 to 13. Further beneficial and preferred embodiments of the present invention are disclosed in the enclosed dependent claims and also described in the following.

A process (i.e. item 1) according to the present invention for producing a triazine ring containing compound, wherein the process comprises an addition reaction between a compound containing a triazine ring and at least two amino groups, and a Michael-acceptor, in the presence of a radical polymerization inhibitor, to obtain an addition product.

Such a process is simple, cheap, does neither require complicated equipment, a solvent, a catalyst nor regeneration steps of reagents and avoids the use of hazardous compounds. In such a process the radical polymerization inhibitor decreases the homopolymerisation of a Michael-acceptor (for details see: J. Therm. Anal. Calorim. DOI 10.1007/s10973-016-5985-6)*.*

The process (i.e. item 2) according to the present invention (i.e. according to item 1), wherein the process additionally comprises an esterification, transesterification, amidation or transamidation reaction between the addition product and a polyfunctional alcohol or a primary or secondary amino alcohol.

Such a process provides easy access to triazine ring containing polyols without (i) the requirement of special equipment and reagent regeneration steps, (ii) the use of hazardous compounds such as formaldehyde, (iii) high process costs due to multistep syntheses, expensive reagents or solvents, and sophisticated purification steps of the final product, (iv) or without that the final products have a short shelf life.

According to another preferred embodiment (i.e. item 3) of the process (i.e. according to item 1 or 2), the addition reaction is preferably a Michael addition reaction.

Such a process ensures the desired chemoselectivity of the addition reaction.

According to another preferred embodiment (item 4) of the process (i.e. according to any one of items 1 to 3), the Michael-acceptor is preferably an unsaturated carboxylic acid with an activated double bond or ester thereof.

Such a process also ensures the desired chemoselectivity of the addition reaction via the presence of an activated double bond in the unsaturated carboxylic acid or ester, a long shelf life and broad applicability of the addition product in obtaining rigid PU/PIR foams having improved flame retardancy properties.

According to another preferred embodiment (i.e. item 5) of the process (i.e. according to item 4), the unsaturated carboxylic acid with an activated double bond or ester thereof is preferably an α,β-unsaturated carboxylic acid or ester thereof.

Such a process also ensures the desired chemoselectivity of the addition reaction, a long shelf life and broad applicability of the addition product in obtaining rigid PU/PIR foams having improved flame retardancy properties.

According to another preferred embodiment (i.e. item 6) of the process (i.e. according to item 4), the unsaturated carboxylic acid with an activated double bond or ester thereof is preferably selected from a group consisting of maleic anhydride, fumaric acid, itaconic acid, and their derivatives.

Such a process provides an addition product of a preferred structure. Here, and also in the following, a preferred structure of an addition product and/or final product ensures a long shelf life and broad applicability thereof in obtaining rigid PU/PIR foams having improved flame retardancy properties.

According to another preferred embodiment (i.e. item 7) of the process (i.e. according to item 6), the α,β-unsaturated carboxylic acid is preferably selected from a group consisting of acrylic acid; alkyl-, cycloalkyl-, aryl- and preferably benzyl-derivatives of acrylic acid; crotonic acid; and alkyl-, cycloalkyl- and benzyl- derivatives of crotonic acids.

Such a process provides an addition product and/or final product of a preferred structure.

According to another preferred embodiment (i.e. item 8) of the process (i.e. according to item 6), the α,β-unsaturated carboxylic ester is preferably selected from a group consisting of methyl-, ethyl-, propyl-, isopropyl, butyl-, pentyl-, hexyl-, phenyl-, benzyl-, and cyclohexylester of α,β-unsaturated carboxylic acid.

Such a process provides an addition product of a preferred structure. The addition product is further kept at short chain length of one molecule of each side chain. Moreover, such a process enables the possibility of removing the released alcohol in a reaction according to item 2.

According to another preferred embodiment (i.e. item 9) of the process (i.e. according to item 6), the α,β-unsaturated carboxylic ester is preferably an ester of a polyfunctional alcohol.

Such a process provides an addition product and/or final product of a preferred structure.

According to another preferred embodiment (i.e. item 10) of the process (i.e. according to item 9), the polyfunctional alcohol preferably is partially or fully esterified.

Such a process provides an addition product and/or final product of a preferred structure. Further, such a process immediately provides a triazine ring containing polyol as an addition product without the need for further reaction according to item 2 in case the polyfunctional alcohol is partially esterified.

According to another preferred embodiment (i.e. item 11) of the process (i.e. according to item 9 or 10), the polyfunctional alcohol is preferably a diol, more preferably a glycol, or a polyglycol.

Such a process provides an addition product and/or final product of a preferred structure.

According to another preferred embodiment (i.e. item 12) of the process (i.e. according to item 9 or 10), the α,β-unsaturated carboxylic ester is preferably selected from the group consisting of hydroxyethyl acrylate, hydroxypropyl acrylate, hydroxyethyl methacrylate, hydroxypropyl methacrylate, 1,3-propane diol mono- and diacrylate, 1,4-butane diol mono- and diacrylate, polyethylene glycol mono- and diacrylate, polyethylene glycol mono- and dimethacrylate, polypropylene glycol mono- and diacrylate, trimethylolpropane triacrylate, and trimethylolpropane-polypropylene glycol triacrylate.

Such a process provides an addition product and/or final product of a preferred structure. Further, such a process immediately provides a triazine ring containing polyol as an addition product without the need for further reaction according to item 2 in case the α,β-unsaturated carboxylic ester is selected from the group consisting of hydroxyethyl acrylate, hydroxypropyl acrylate, hydroxyethyl methacrylate, hydroxypropyl methacrylate.

According to another preferred embodiment (i.e. item 13) of the process (i.e. according to any one of items 1 to 12), the produced triazine ring containing compound is preferably a 1,3,5-triazine ring containing compound, more preferably a 1,3,5-triazine ring containing polyol.

Such a process provides an addition product and/or final product of a preferred structure. Further, such a process provides final products with better flame retardancy properties in comparison to traditional aromatic polyesters while keeping the general properties like viscosity or hydroxyl value similar.

According to another preferred embodiment (i.e. item 14) of the process (i.e. according to any one of items 1 to 13), the triazine compound containing at least two amino groups is preferably selected from the group consisting of melamine, mono- or polyalkyl substituted derivatives of melamine, mono- or polyacyl substituted derivatives of melamine, imido melamine derivatives, aryl- or alkyl guanamines, alkyl or aryl substituted derivatives of guanamines, and products of partial hydrolysis of melamine, such as ammeline.

Such a process provides an addition product and/or final product of a preferred structure.

According to another preferred embodiment (i.e. item 15) of the process (i.e. according to any one of items 1 to 14), the radical polymerization inhibitor is preferably selected from the group consisting of hydrochinone, phenothiazine and paramethoxyphenol.

Such a process ensures the essential inhibition of a radical polymerization of the Michael-acceptor, which proceeds especially at elevated temperatures, at reasonably low inhibitor concentrations.

According to another preferred embodiment (i.e. item 16) of the process (i.e. according to any one of items 1 to 15), the amount of the radical polymerization inhibitor is preferably within the range of 0.1 to 0.5 mol% with respect to the triazine compound containing at least two amino groups, more preferably in the range of 0.15 to 0.25 mol%.

By choosing such a process, the residual amount of polymerization inhibitor in the final product is reasonably low.

According to another preferred embodiment (i.e. item 17) of the process (i.e. according to any one of items 6 to 16), the molar ratio of α,β-unsaturated carboxylic acid or ester thereof to the compound containing a triazine ring and at least two amino groups is preferably within the range from 1:1 to 20:1, more preferably within the range from 3:1 to 6:1, even more preferably 2:1 to 4:1.

Such a process provides an addition product and/or final product with a desired side chain length of one to three repetition units (i.e. e.g. n = 0, 1, or 2). Thus, the content of the triazine compound containing at least two amino groups in the final polyol is high, which leads to improved flame retardancy properties of a flame retardant.

According to another preferred embodiment (i.e. item 18) of the process (i.e. according to any one of items 1 to 17), the addition product is preferably a molecule (i.e. a triazine ring containing polyester polyol) according to Fig. 1, i.e. according to the following general formula (1), 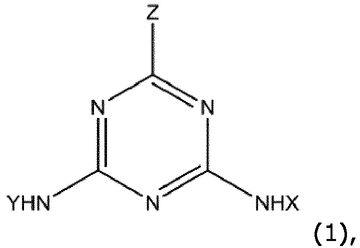 wherein X is represented by -CH(R₁)CH(R₂)COOR₃ where R₁ and R₂ are each independently a hydrogen atom, a hydrocarbon group, a carboxylic group or derivative thereof, or a methylene carboxylic group or derivative thereof, R₃ is selected from the group consisting of a hydrogen atom, a hydrocarbon group, a structural element that forms a cyclic structure with R₁, such as an anhydride, and a linear fragment of structure (i):

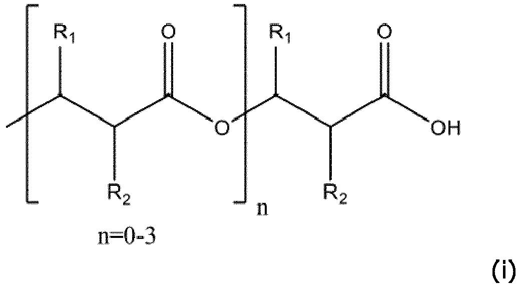

Y is represented by one of the groups corresponding to X, Z is selected from a group consisting of a hydrocarbon group, -OR₄, -NHR₅, and an imido group, where R₄ is a hydrogen atom, or a hydrocarbon group, R₅ is selected from a group consisting of a hydrogen atom, a hydrocarbon group, an acyl group, or one of the groups corresponding to X.

Such a process provides addition products suitable for further transformation according to item 2 in order to obtain triazine ring containing polyols. In such a process n strongly depends on the molar excess of the Michael acceptor.

According to another preferred embodiment (i.e. item 19) of the process (i.e. according to item 18), the hydrocarbon group is preferably selected from the group consisting of an alkyl group, an aryl group, a cycloalkyl group, a benzyl group, an alkenyl group, a cycloalkenyl group, and an arylalkyl group.

Such a process provides an addition product and/or final product of a preferred structure.

According to another preferred embodiment (i.e. item 20) of the process (i.e. according to item 18), the hydrocarbon group is preferably selected from the group consisting of an alkyl group, an aryl group, a cycloalkyl group, and a benzyl group.

Such a process provides an addition product and/or final product of a preferred structure.

According to another preferred embodiment (i.e. item 21) of the process (i.e. according to any one of items 1 to 20), the overall process, additionally comprising an esterification, transesterification, amidation or transamidation reaction between the addition product and a polyfunctional alcohol or a primary or secondary amino alcohol, is preferably a one or two-step process.

Such a process provides economical benefits such as savings of time and resources.

According to another preferred embodiment (i.e. item 22) of the process (i.e. according to any one of items 2 to 21), the reaction temperature in a one step process is preferably within the range from 130°C to 230°C, more preferably within the range from 170°C to 200°C.

Such a process decreases side reactions, such as hydrolysis of amine groups, glycol cyclization under water formation and etherification of glycols, and the amount of side products, thus yielding more and purer polyols. For example, such side reactions have been found to be decomposition processes followed by ammonia or amine release.

In this context a one step process is defined as a process, wherein the addition reaction according to item 1 and the esterification-, transesterification-, amidation- or transamidation- (i.e. in the following referred to as "(trans)esterification/(trans)amidation") reaction according to item 2 proceed simultaneously. Respectively, a two step process is defined as a process, wherein the addition reaction and the (trans)esterification/(trans)amidation occur subsequently.

According to another preferred embodiment (i.e. item 23) of the process (i.e. according to any one of items 1 to 21), the reaction temperature of the addition reaction in a two step process is preferably within the range from 90°C to 130°C.

Such a process decreases side reactions and the amount of side products, thus yielding more and purer addition products.

According to another preferred embodiment (i.e. item 24) of the process (i.e. according to item 22 or 23), the normal boiling point of the corresponding alcohol obtainable from alcoholysis of the α,β-unsaturated carboxylic ester is preferably lower than the reaction temperature.

Such a process allows the corresponding alcohol obtainable from alcoholysis of the α,β-unsaturated carboxylic ester to be removed from the reaction mixture during esterification or transesterification.

The exception from this rule are the esters with polyfunctional alcohols according to item 9 which shouldn't be removed from the reaction. In this certain case only the Michael addition of a compound containing a triazine ring and at least two amino groups and a Michael-acceptor has to be carried out, no transesterification is needed in order to obtain the final polyol.

According to another preferred embodiment (i.e. item 25) of the process (i.e. according to any one of items 2 to 24), the process preferably is carried out at atmospheric pressure or under vacuum.

Such a process allows to keep the reaction temperature in a preferred range while at the same time removing the volatile components of the reaction mixture. In the present invention the term atmospheric pressure relates to a value of 100 kPa according to IUPAC recommendations and the term vacuum relates to a value within the range of 25 to 750 mmHg.

According to another preferred embodiment (i.e. item 26) of the process (i.e. according to any one of items 1 to 25), a catalyst for the addition reaction is preferably used.

Such a process enables shorter reaction times and lower reaction temperatures. Further, the use of a catalyst also provides higher selectivity and fewer side reactions, thus yielding polyols in higher yields and higher purity.

According to another preferred embodiment (i.e. item 27) of the process (i.e. according to item 26), the catalyst preferably is a Brǿnsted base and/or a Lewis acid.

In general, either Lewis acids or Brǿnsted bases can catalyze both (trans)esterification/(trans)amidation reactions and addition reactions to an activated double bond. However, for the described process the combination of both was found to give the best results regarding lower reaction temperatures, shorter reaction times, better colour of the final product, and less losses through side reactions.

According to another preferred embodiment (i.e. item 28) of the process (i.e. according to item 27), the Brǿnsted base is preferably selected from a group consisting of hydroxides, alcoholates, salts of alkali or alkali earth metals, and tertiary amines, and the amount of the Brǿnsted base in said process is more preferable in the range of 0.1 to 1%, even more preferable in the range of 0.2 to 0.5%.

Such a process enables shorter reaction times and lower reaction temperatures with low loadings of catalyst, thus yielding more and purer polyols.

According to another preferred embodiment (i.e. item 29) of the process (i.e. according to item 27), the Lewis acid is preferably selected from a group consisting of chlorides and organic derivatives of tin (II, IV), of zinc, of aluminum, of led, of iron (III), and of titanium (IV).

Such a process enables shorter reaction times and lower reaction temperatures with low loadings of catalyst, thus yielding more and purer polyols.

According to another preferred embodiment (i.e. item 30) of the process (i.e. according to any one of items 1 to 29), preferably no catalyst for the addition reaction is used.

Generally, catalyst residues in the final product impact the reactivity of the final product and can create some problems to the users. In case catalyst residues are not tolerated, an additional purification or catalyst removal step must be implemented in the process. This purification step includes neutralization of the base, crystallization and filtration and is usually applied for the production of polyether polyols for application mainly in polyurethane (PU) foams. Therefore, the presented process avoids the above mentioned need for additional purification steps.

According to another preferred embodiment (i.e. item 31) of the process (i.e. according to any one of items 1 to 30), preferably no regeneration step of reagents used in the process is required.

Such a process provides economical benefits.

According to another preferred embodiment (i.e. item 32) of the process (i.e. according to any one of items 1 to 31), preferably no solvent is implemented in the process.

Such a process provides economical benefits.

According to another preferred embodiment (i.e. item 33) of the process (i.e. according to any one of items 1 to 32), the triazine ring containing polyol has preferably a hydroxyl value determined by substitutional titration according to ISO 14900:2017 within the range of 180-300 mgKOH/g or within the range of 300-800 mgKOH/g.

Such a process provides a triazine ring containing polyol that is well applicable for PIR foams with improved fire retardancy properties when the above hydroxyl value is within the range of 180-300 mgKOH/g and well applicable for PU foams when the above hydroxyl value is within the range of 300-800 mgKOH/g.

According to another preferred embodiment (i.e. item 34) of the process (i.e. according to any one of items 1 to 33), the triazine ring containing compound has preferably a dynamic viscosity determined according to ISO 3219:1993 within the range of 500-50000 mPa·s, more preferably within the range of 1500-30000 mPa·s, and even more preferably within the range of 2000-10000 mPa·s.

Such a process provides a triazine ring containing polyol which is easier to process when used for PIR foams with improved fire retardancy properties.

According to another preferred embodiment (i.e. item 35) of the process (i.e. according to any one of items 1 to 34), a polyester polyol is preferably obtained by esterification of a carboxylic group of the addition product.

Such a process is able to provide a final product with a side chain length larger than one repetition unit.

According to another preferred embodiment (i.e. item 36) of the process (i.e. according to any one of items 1 to 34), a polyester polyol is preferably obtained by transesterification of an ester group of the addition product.

Such a process provides a final product with a side chain length equal to one repetition unit.

According to another preferred embodiment (i.e. item 37) of the process (i.e. according to any one of items 1 to 34), an amido polyol is preferably obtained by amidation of carboxylic groups of the addition product.

Such a process is able to provide a final product with a side chain length larger than one repetition unit.

According to another preferred embodiment (i.e. item 38) of the process (i.e. according to any one of items 1 to 34), an amido polyol is preferably obtained by transamidation of an ester group of the addition product.

Such a process provides a final product with a side chain length equal to one repetition unit.

According to another preferred embodiment (i.e. item 39) of the process (i.e. according to any one of items 6 to 38), the amount of polyfunctional alcohol or amino alcohol is preferably equimolar to the amount of α,β-unsaturated carboxylic acid or ester thereof.

Such a process enables an easier (trans)esterification or (trans)amidation process.

According to another preferred embodiment (i.e. item 40) of the process (i.e. according to any one of items 2 to 39), the polyfunctional alcohol is preferably selected from a group consisting of ethylene-, diethylene-, thioethylene and higher polyethylene glycols, propylene-, dipropylene-, tripropylene- and higher propylene glycols, 1,4-, 1,3-, 1,2-butandiols, polytetramethylendiols, 1,3-propane diol, neopentylglycol, 1,5-pentane- and 1,6-hexane-diols, glycerin, hexane triol, thrimethylol propane, thrimethylol ethane, pentaerythritol, dipentaerrythritol, and alkoxylation derivatives thereof.

Such a process provides a final product of a preferred structure.

According to another preferred embodiment (i.e. item 41) of the process (i.e. according to any one of items 2 to 40), the amino alcohol is preferably selected from a group consisting of ethanolamine, isopropanol amine, 2-aminoethanol, 1-amino-3-ethanol, n-butyl amino ethanol, n-propyl amino ethanol, and tert-butyl amino ethanol.

Such a process provides a final product of a preferred structure.

According to another preferred embodiment (i.e. item 42) of the process (i.e. according to any one of items 2 to 41), a modifier is preferably used.

Such a process achieves improvement of specific properties of the addition product or final product. In the case of polyols used in PU/PIR foams such properties are fire retardancy, viscosity, surface tension or compatibility with blowing agents.

According to another preferred embodiment (i.e. item 43) of the process (i.e. according to item 42), the modifier is preferably a monoalcohol or ether thereof, or a carboxylic acid, -ester thereof, or -anhydride thereof. More preferably, if the modifier is a monoalcohol, the normal boiling point of the monoalcohol is 150°C or higher.

Such a process (e.g. using of long chain monofunctional alcohols or ethers thereof, carboxylic acids, -esters thereof or -anhydrides thereof) results in the reduction of viscosity and improvement of the compatibility with blowing agents, in particular the compatibility with non-polar blowing agents, such as pentane, isopentane, cyclopentane, and their mixtures, and fluorinated blowing agents.

According to another preferred embodiment (i.e. item 44) of the process (i.e. according to item 43), the monoalcohol is preferably a C7-alcohol or higher such as heptanol, octanol, 2-ethylhexanol, lauryl alcohol, stearyl alcohol, and the ether of a monoalcohol is an alkoxylation product of alcohols including lower than C6-alcohols with alcohols such as methanol, propanol, isopropanol, butanol, isobutanol or higher alcohols, or an addition product of alcohols including lower than C6-alcohols with alkylene oxides such as ethylene oxide, propylene oxide, butylene oxide or higher oxides.

Such a process achieves a similar further improved effect as mentioned under item 43. Further, when ethoxylation products of monoalcohols are used, the surface tension can be reduced and the emulsifying ability of the polyol is improved.

In case of using emulsion technology for the production of PU/PIR foams, the production preferably is typically a continuous production of the insulation boards with flexible or rigid facings.

According to another preferred embodiment (i.e. item 45) of the process (i.e. according to item 43), the carboxylic acid, ester thereof, or anhydride thereof is preferably selected from a group consisting of monocarboxylic acids such as 2-ethylhexanoic acid, versatic acid and fatty acids, polycarboxylic acids such as adipic-, glutaric-, succinic-, sebacic-, azelaic-, phthalic-, terephtalic-, isophtalic- and hexahydrophtalic acid, anhydrides such as phtalic-, hexahydrophtalic-, succinic-, alkylsuccinic- and maleic anhydride, natural oils, and methyl esters of natural oil fatty acids, such as methyl esters of rapeseed oil.

The modifiers defined in this paragraph are used for the production of typical polyesterpolyols. Said process allows the combination of the present invention with classical modifies in one process.

According to another preferred embodiment (i.e. item 46) of the process (i.e. according to any one of items 2 to 45), an acid scavenger is preferably used.

Such a process provides a final product with a low acid value.

According to another preferred embodiment (i.e. item 47) of the process (i.e. according to item 46), the acid scavenger is preferably selected from a group consisting of cyclic carbonates, such as ethylene-, propylene-, glycerin carbonates, or epoxy group containing compounds, such as alkylene oxides, preferably ethylene-, propylene-, butylene oxides, styrene oxides, epoxidized higher olefins, epoxidized natural oils, such as epoxidized soybean oil or line seed oil, glycidol and its derivatives, such as 2-ethylhexyl glycidyl ether, glycidyl esters of pivalic-, versatic acid, and 2-ethyl hexanoic acid.

Such a process provides a final product with a low acid value.

An addition product (i.e. item 48) is obtained by the addition reaction in the process as described herein (i.e. any one of items 1 to 47).

Such an addition product is particularly suitable for further transformation into a triazine ring containing polyol via a process according to one of items 2 to 47.

According to the present invention, use (i.e. item 49) of the addition product as described herein (i.e. item 48) for obtaining a triazine ring containing compound by esterification, transesterification, amidation or transamidation reaction between the addition product and a polyfunctional alcohol or an amino alcohol is made.

Such use enables the formation of a final product particularly useful for producing PU/PIR foams with improved fire retardancy properties.

According to the present invention, a triazine ring containing polyol (i.e. item 50) is obtained by a process as described herein (i.e. according to any one of items 1 to 47).

Such a compound is particularly stable ensuring a long shelf life. Further, due to the particularly short chain lengths of the side chains, the hydroxyl value and viscosity of these polyols are particularly efficient when used in rigid PU/PIR foams and able to improve their flame retardancy properties. An additional advantage of the polyols is that they allow to easily achieve a desired combination of viscosity and hydroxyl value, which makes them well applicable either for PU or PIR rigid foams with improved fire retardancy properties.

According to the present invention, use (i.e. item 51) of the triazine ring containing polyol as described herein (i.e. item 50) obtained by a process as described herein (i.e. according to any one of items 1 to 47) for producing PU/PIR foams is made.

Such a use provides PU/PIR foams, in particular PU/PIR rigid foams, with improved fire retardancy properties.

In this regard, it is preferable that a triazine ring containing polyol has a functionality (i.e. alcohol functionality, which corresponds to reactive alcohol groups of the triazine ring containing polyol) of no less than 3, more preferably in the range of 3 to 8, when being used for PU foams and of no less than 2, even more preferably in the range of 2 to 3, when being used for PIR foams. Such a functionality may be influenced by the initial ratio between the Michael-Acceptor and a triazine ring containing compound having at least two amino groups. Further, such a functionality may be influenced by the polyfunctional alcohol or primary or secondary amino alcohol in a (trans)esterification/(trans)amidation reaction.

According to the present invention, a PU/PIR (i.e. PU or PIR) foam (i.e. item 52) is obtained by using the triazine ring containing polyol as described herein (i.e. item 50).

Such a PU/PIR foam provides improved fire retardancy properties. PU/PIR foams in the present invention refer preferably to PU/PIR foams as defined by the *"Verband für Dämmsysteme, Putz und Mörtel e.V."(cf. e.g.* "Qualitätsriehtlinien für Dämmstoffe zur Verwendung in Wärmedämm-Verbundsystemen (WDVS) aus Polyurethan-Hartschaum", 2. Auflage, Stand: Januar 2020). Rigid PU/PIR foams preferably have a compression strength as determined according to DIN EN 826 of equal to or greater than 100 kPa (cf. also the above cited document of the *"*Verband für Dämmsysteme, Putz und Mortel e. V.", bullet point 4.6*).*

### Detailed description of preferred embodiments

### EXAMPLES

Hereinafter, examples of the present invention will be described. Incidentally, the present invention is not limited to the following examples.

### Example 1:

To the solution of 1.3 g para-methoxyphenol (0.01 mol) representing a polymerization inhibitor in 346.4 g (4.8 mol) water-free acrylic acid representing an α,β-unsaturated carboxylic acid 109.8 g (0.87 mol) melamine representing a compound containing a triazine ring and at least two amino groups were added portion wise in a four necked glass flask equipped with a nitrogen inlet, an agitator, a distillation column and a temperature control. The temperature was slowly increased to 130°C until a clear viscous solution was formed. After approximately 15 min of agitating 389.5 g (5.1 mol) propylene glycol representing a polyfunctional alcohol and 0.2 g (0.6 mmol) tetrabutyltitanate representing a Lewis acid were added to the reaction mixture and the esterification process under water removal was carried out at 185-200°C approximately 8 hours. The final dark brown liquid product provided the following properties:
Dynamical viscosity at 25°C: 54600 mPa·s, acid value: 3.5 mgKOH/g; water content: 0.15%; hydroxyl value: 457 mgKOH/g, iodine value: 2.5.

### Example 2:

To the suspension of 260 g (2.06 mol) melamine representing a compound containing a triazine ring and at least two amino groups, 4 g NaOH (0.1 mol) representing a Brǿnsted base and 2 g hydroquinone (0.018 mol) representing a polymerization inhibitor in 1300 g PEG200 (6,5 mol) and 180 g propylene glycol (2,37 mol) representing a combination of polyfunctional alcohols at 70°C, 440 g water-free acrylic acid (6.1 mol) representing an α,β-unsaturated carboxylic acid were slowly added under agitation in the four necked glass flask equipped with the nitrogen inlet, the agitator, the distillation column and the temperature control. After addition of acrylic acid, the temperature was slowly increased to 140°C and 0.4 g (0.0011 mol) tetrabutyltitanate representing a Lewis acid were added to the system. After that, the temperature was slowly increased to 180°C and esterification under water removal was carried out (app. 8 hours). The system became clear after 4 hours of agitating at 180°C. The final dark amber liquid product provided the following properties:
Dynamical viscosity at 25°C: 7200mPa·s, acid value: 1.8 mgKOH/g; water content: 0.14%; hydroxyl value: 258.4 mgKOH/g, iodine value: 3.1.

### Example 3:

To the suspension of 260 g (2.06 mol) melamine representing a compound containing a triazine ring and at least two amino groups 4 g (0.1 mol) NaOH representing a Brǿnsted base and 2 g (0.015 mol) para-methoxy phenol representing a polymerization inhibitor in 300 g (4.98 mol) propylene glycol representing a polyfunctional alcohol at 70°C, 440 g (6.1 mol) water-free acrylic acid representing an α,β-unsaturated carboxylic acid were slowly added under agitation in the four necked glass flask equipped with the nitrogen inlet, the agitator, the distillation column and the temperature control. After addition of melamine, the temperature was slowly increased to 140°C and 0.8 g (0.0011 mol) tetrabutyltitanate representing a Lewis acid were added to the system. After addition of tetrabutyltitanate the temperature was slowly increased to 180°C and kept at this temperature under water removal until a clear solution was formed (app. 4 hours) and the acid value of the system was in the range 10-15 mgKOH/g. After addition of 100 g (0.98 mol) propylene carbonate representing an acid scavenger the reaction mixture was further agitated at 180°C another two hours and stopped. The final dark amber product provided the following properties:
Dynamical viscosity at 25°C: 13200 mPa·s, acid value: 2.18 mgKOH/g; water content: 0.13%; hydroxyl value: 222 mgKOH/g, iodine value: 3.2.

### Example 4:

To the solution of 2 g (0.05 mol) NaOH representing a Brǿnsted base and 1 g (0.0075 mol) para-methoxy phenol representing a polymerization inhibitor in 195 g (1.68 mol) hydroxyethyl acrylate representing an ester of a α,β-unsaturated carboxylic acid and 87.5 g (0.22 mol) of PEG400 representing a polyfunctional alcohol, 65 g (0.515 mol) melamine representing compound containing a triazine ring and at least two amino groups have been added at 60°C. After addition, the temperature was slowly increased to 180°C and kept at this level until the reaction mixture became clear (app. 12 hours). The final amber product provided the following properties:
Dynamical viscosity at 25°C: 86100 mPa·s, acid value: 0.14 mgKOH/g; water content: 0.05%; hydroxyl value: 330 mgKOH/g, iodine value: 1.8.

### Measuring conditions:

The following measuring conditions of the properties of the final polyols are described below under specification of the respective international standard:
The hydroxyl value was determined by substitutional titration according to the standard test method ASTM D4274, which fulfils the requirements of ISO 14900:2017)
The acid value was determined by direct titration according to the standard test method ASTM D 4662, which fulfils the requirements of ISO 2114:2000.

The dynamical viscosity at 25°C was determined by a Brookfield test according to the standard test method ASTM D4878, which fulfils the requirements of ISO 3219:1993.

The iodine value was determined by the Wijs method according to the standard test method ASTM D 4671.

The water content was determined by Karl Fischer titration according to the standard test method ASTM D 4672, which fulfils the requirements of ISO 14897:2002.

The herein described preferred embodiments can be combined freely to describe more preferred embodiments. In this regard, the combinations derivable from the combination of items indicated in brackets are by no means limiting the preferred embodiments and merely indicate particularly more preferable combinations of preferred embodiments.

The present invention relates to a process for producing a triazine ring containing compound, wherein the process comprises an addition reaction between a compound containing a triazine ring and at least two amino groups, and a Michael-acceptor, in the presence of a radical polymerization inhibitor, to obtain an addition product, wherein the process additionally comprises an esterification, transesterification, amidation or transamidation reaction between the addition product and a polyfunctional alcohol or a primary or secondary amino alcohol.

## Claims

1. A process for producing a triazine ring containing compound, wherein the process comprises:
an addition reaction between a compound containing a triazine ring and at least two amino groups, and a Michael-acceptor, in the presence of a radical polymerization inhibitor, to obtain an addition product, wherein the process additionally comprises an esterification, transesterification, amidation or transamidation reaction between the addition product and a polyfunctional alcohol or a primary or secondary amino alcohol.

2. The process according to claim 1, wherein the Michael-acceptor is an unsaturated carboxylic acid with an activated double bond or ester thereof.

3. The process according to claim 2, wherein the unsaturated carboxylic acid with an activated double bond or ester thereof is an α,β-unsaturated carboxylic acid or ester thereof, preferably one selected from the group consisting of acrylic acid; alkyl-, cycloalkyl-, aryl- and preferably benzyl-derivatives of acrylic acid; crotonic acid; and alkyl-, cycloalkyl- and benzyl- derivatives of crotonic acids.

4. The process according to any one of claims 1 to 3, wherein the compound containing a triazine ring and at least two amino groups is selected from the group consisting of melamine, mono- or polyalkyl substituted derivatives of melamine, mono- or polyacyl substituted derivatives of melamine, imido melamine derivatives, aryl- or alkyl guanamines, alkyl- or aryl substituted derivatives of guanamines, and products of partial hydrolysis of melamine, such as ammeline, preferably melamine.

5. The process according to any one of claims 1 to 4, wherein the molar ratio of the Michael-acceptor to the compound containing a triazine ring and at least two amino groups is within the range from 1:1 to 20:1, preferably within the range from 3:1 to 6:1, more preferably 2:1 to 4:1.

6. The process according to any one of claims 1 to 5, wherein the addition product is a molecule according to the following general formula (1), 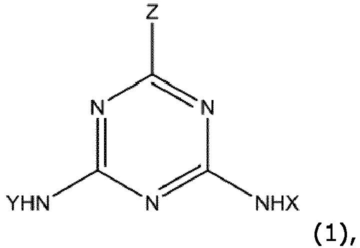 wherein
X is represented by -CH(R₁)CH(R₂)COOR₃
where
R₁ and R₂ are each independently a hydrogen atom, a hydrocarbon group, a carboxylic group or derivative thereof, or a methylene carboxylic group or derivative thereof,
R₃ is selected from the group consisting of a hydrogen atom, a hydrocarbon group, a structural element that forms a cyclic structure with R₁ and a linear fragment of structure (i): 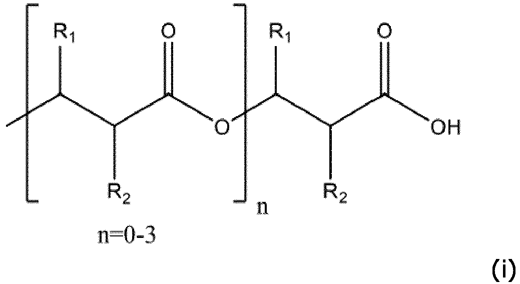
Y is represented by one of the groups corresponding to X,
Z is selected from a group consisting of a hydrocarbon group, -OR₄, -NHR₅, and an imido group, where
R₄ is a hydrogen atom, or hydrocarbon group,
R₅ is selected from a group consisting of a hydrogen atom, a hydrocarbon group, an acyl group, or one of the groups corresponding to X.

7. The process according to any one of claims 1 to 6, wherein the overall process is a one or two-step process optionally comprising one of the following:
(i) the reaction temperature in a one step process is within the range from 130°C to 230°C, preferably within the range from 170°C to 200°C, or
(ii) the reaction temperature of the addition reaction in a two step process is within the range from 90°C to 130°C.

8. The process according to any one of claims 1 to 7, wherein a catalyst is used.

9. The process according to any one of claims 1 to 8, wherein the by the process obtained triazine ring containing polyol has a hydroxyl value determined by substitutional titration according to ISO 14900:2017 within the range of 180-300 mgKOH/g or within the range of 300-800 mgKOH/g and/or wherein the by the process obtained triazine ring containing polyol has a dynamic viscosity determined according to ISO 3219:1993 within the range of 500-50000 mPa·s, preferably within the range of 1500-30000 mPa·s, and more preferably within the range of 2000-10000 mPa·s.

10. A triazine ring containing polyol obtained by a process according to any one of claims 1 to 9, wherein the addition product is as defined in claim 6.

11. Use of the addition product obtained by the addition reaction as defined in any one of claims 1 to 9 for obtaining a triazine ring containing polyol by esterification, transesterification, amidation or transamidation reaction between the addition product and a polyfunctional alcohol or a primary or secondary amino alcohol.

12. Use of the triazine ring containing polyol according to claim 10 for producing a PU/PIR foam.

13. PU/PIR foam obtained by using the triazine ring containing polyol according to claim 10.
